# FASCICULE DE BREVET EUROPEEN

(11) **EP 3 823 657 B1**
(45) Date de publication et mention de la délivrance du brevet: **05.06.2024**
(21) Numéro de dépôt: 19742048.2
(22) Date de dépôt: 19.07.2019
(51) Int. Cl.: A61K 38/01, A61K 8/64, A61K 9/00, A61P 17/00, A61P 17/08, A61P 17/02, A61K 31/198, A61K 31/122, A61K 38/40, A61K 38/20, A61Q 19/08

(54) **COMPOSITIONS A USAGE COSMETIQUE ET DERMATOLOGIQUE**
ZUSAMMENSETZUNGEN FÜR KOSMETISCHE UND DERMATOLOGISCHE VERWENDUNG
COMPOSITIONS FOR COSMETIC AND DERMATOLOGICAL USE

(30) Priorité: 20.07.2018 FR 1856756
(43) Date de publication de la demande: 26.05.2021
(73) Titulaire: Semiocare SAS, 75005 Paris (FR)
(72) Inventeur: CAPPELLO, Serge, 93290 Tremblay en France (FR)
(74) Mandataire: Bandpay & Greuter
(86) Numéro de dépôt international: PCT/EP2019/069603
(87) Numéro de publication internationale: WO 2020/016450

(56) Documents cités:
- EP-A1- 1 997 477
- WO-A1-2004/047566
- WO-A1-2006/098625
- WO-A1-2008/056983
- WO-A1-2015/125067
- WO-A2-99/65329
- CA-A1- 2 580 372
- FR-A1- 2 781 150
- JP-A- H03 220 130
- US-A1- 2013 089 572
- AHMED SAMIR EDRISS ET AL: "Therapy of keloid and hypertrophic scars: a review", EUROPEAN JOURNAL OF PLASTIC SURGERY, SPRINGER, BERLIN, DE, vol. 34, no. 6, 1 juillet 2011 (2011-07-01), pages 425-436, XP019978542, ISSN: 1435-0130, DOI: 10.1007/S00238-011-0602-1
- CALLEN J ET AL: "A systematic review of the safety of topical therapies for atopic dermatitis.", THE BRITISH JOURNAL OF DERMATOLOGY FEB 2007, vol. 156, no. 2, février 2007 (2007-02), pages 203-221, XP055564581, ISSN: 0007-0963
- MICLO L ET AL: "Characterization of alph-casozepine, a tryptic peptide from bovine alphas1-casein with benzodiazepine-like activity", THE FASEB JOURNAL, FEDERATION OF AMERICAN SOCIETIES FOR EXPERIMENTAL BIOLOGY, US, vol. 15, no. 10, 1 August 2001 (2001-08-01), pages 1780-1792, XP002481109, ISSN: 0892-6638 [retrieved on 2001-06-08]

## Description

### DOMAINE DE L'INVENTION

La présente invention concerne des nouvelles compositions utiles dans le traitement cosmétique de la peau ou dans le traitement de maladies dermatologiques.

### ARRIERE-PLAN TECHNIQUE

Des propriétés de nombreux composés d'origine animale ou végétale pouvant être bénéfiques pour l'homme ou l'animal ont été mises en évidence.

Par exemple, le document de Jean Soon Park et al., Astaxanthin decreased oxidative stress and inflammation and enhanced immune response in humans, Nutr Metab (Lond), 2010, 7:18, divulgue qu'un régime alimentaire d'astaxanthine augmente à la fois la réponse immunitaire humorale et la réponse immunitaire à médiation cellulaire chez les jeunes femmes saines.

Le document de P. P. Ward et al., Multifunctional roles of lactoferrin: a critical overview, Cell. Mol. Life Sci., 2005, vol. 62, p.2540-2548, décrit différentes fonctions de la lactoferrine, comme la capture du fer dans l'intestin, une action antimicrobienne, une fonction anti-inflammatoire, une protection contre le cancer et un rôle dans la morphogénèse des os.

Le document de Didier Levieux, Le colostrum, un lait particulièrement riche en de nombreux composants : peut-on en déceler la présence dans les livraisons de lait de vache ?, Lait, 1999, vol.75, p.465-488, décrit les composants du colostrum bovin et le document de Sylvie F. Gauthier et al., Growth factors from bovine milk and colostrum: composition, extraction and biological activities, Lait, 2006, vol. 86, p.99-125 énumère les facteurs de croissance présents dans le colostrum et dans le lait et décrit leurs propriétés.

Cependant, les propriétés bénéfiques de ces composants sont connues essentiellement dans le cas d'une prise alimentaire.

Le document JP H03-220130 décrit un produit inhibant l'implantation d'agents pathogènes, comprenant de la lactoferrine ou un produit de dégradation de celle-ci obtenu avec une protéase, et de la K-caséine ou un produit de décomposition de celle-ci obtenu avec une protéase.

Pour une variété d'applications cosmétologiques et dermatologiques, il existe un besoin de fournir une composition permettant de prolonger la durée de vie des cellules de l'épiderme afin, notamment, de ralentir l'atrophie cutanée.

### RESUME DE L'INVENTION

L'invention concerne en premier lieu une composition comprenant une glycoprotéine liant le fer et un hydrolysat de la caséine qui est l'α-casozépine. Le ratio massique de la glycoprotéine liant le fer par rapport à l'α-casozépine est de 0,5 à 3

Dans des modes de réalisation, la glycoprotéine liant le fer est la lactoferrine.

Dans des modes de réalisation, la composition comprend en outre :
- du colostrum, de préférence du colostrum bovin, du colostrum ovin, du colostrum équin ou du colostrum porcin, encore plus préférentiellement du colostrum bovin ; et / ou
- un précurseur de l'oxyde nitrique, de préférence de la L-arginine ;
- un antioxydant, de préférence un caroténoïde, plus préférentiellement de l'astaxanthine ; et/ou
- de la sérotonine ou un de ses précurseurs, qui de préférence est le tryptophane.

Dans des modes de réalisation, la composition comprend, en poids de matière sèche :
- de 2 à 30 %, de préférence de 10 à 30 % de glycoprotéine liant le fer ; et/ou
- de 2 à 30 %, de préférence de 3 à 20 % d'hydrolysat de caséine ; et/ou
- de 15 à 70 %, de préférence de 20 à 50 % de colostrum ; et/ou
- de 10 à 50 %, de préférence de 15 à 40 % de précurseur d'oxyde nitrique ; et/ou
- de 2 à 30 %, de préférence de 3 à 20 % d'antioxydant ; et/ou
- de 2 à 30 %, de préférence de 3 à 20 % de sérotonine ou précurseur de celle-ci.

Dans des modes de réalisation, la composition comprend, en poids de matière sèche :
- de 2 à 30 %, de préférence de 10 à 30 % de lactoferrine ; et/ou
- de 2 à 30 %, de préférence de 3 à 20 % d'α-casozépine ; et/ou
- de 15 à 70 %, de préférence de 20 à 50 % de colostrum ; et/ou
- de 10 à 50 %, de préférence de 15 à 40 % de L-arginine ; et/ou
- de 2 à 30 %, de préférence de 3 à 20 % d'astaxanthine ; et/ou
- de 2 à 30 %, de préférence de 3 à 20 % de tryptophane.

Dans des modes de réalisation, la composition comprend, en poids de matière sèche :
- de 15 à 20 % de lactoferrine ;
- de 5 à 10 % d'α-casozépine ;
- de 25 à 45 % de colostrum ;
- de 20 à 30 % de L-arginine ;
- de 5 à 10 % d'astaxanthine ; et
- de 5 à 10 % de tryptophane.

L'invention concerne également un procédé de traitement cosmétique de la peau, comprenant l'application topique d'une composition telle que décrite ci-dessus.

Dans des modes de réalisation, le traitement cosmétique de la peau est le comblement des rides, la diminution des poches sous les yeux, la réduction de la couleur des cernes, la réduction des tâches de vieillissement, l'augmentation de l'élasticité de la peau et/ou l'atténuation des vergetures.

L'invention concerne également la composition telle que décrite ci-dessus, pour son utilisation dans le traitement des maladies dermatologiques.

Dans des modes de réalisation, la composition est destinée à être utilisée dans le traitement de l'atrophie cutanée, l'eczéma et/ou les chéloïdes.

La présente invention permet de répondre au besoin ci-dessus. Elle fournit plus particulièrement une composition permettant d'augmenter la taille des kératinocytes. Ainsi, la composition selon l'invention permet de renforcer les cellules de la peau, de retarder leur dégénérescence et donc d'augmenter leur durée de vie.

Cela est accompli principalement grâce à l'association de deux composés : une glycoprotéine liant le fer et un hydrolysat de la caséine ; et dans des modes de réalisation préférés grâce à l'association de ces composés avec des composés supplémentaires.

### DESCRIPTION DE MODES DE REALISATION DE L'INVENTION

L'invention est maintenant décrite plus en détail et de façon non limitative dans la description qui suit.

Dans la présente description, sauf indication expresse différente, tous les pourcentages (%) indiqués sont des pourcentages en poids.

Dans la présente demande, le terme « *une glycoprotéine liant le fer* » doit être compris comme signifiant « *une ou plusieurs glycoprotéines liant le fer* » et « *hydrolysat de la caséine* » doit être compris comme signifiant « *un ou plusieurs hydrolysats de la caséine* ». Il en est de même pour les autres composés de la composition.

### Composition

L'invention concerne une composition comprenant une glycoprotéine liant le fer (composé A) et un hydrolysat de la caséine (composé B).

De préférence, la glycoprotéine liant le fer est la lactoferrine. La lactoferrine est une protéine présente dans le lactosérum de lait, par exemple du lait humain ou bovin. La lactoferrine est une glycoprotéine multifonctionnelle jouant un rôle dans la préservation des cellules contre les agressions intrinsèques. Elle affecte la prolifération, la maturation et l'activation de plusieurs types de cellules immunitaires et a un rôle dans la régulation, la maturation et l'activation des neutrophiles et des macrophages, et dans la maturation et la fonction des lymphocytes. La lactoferrine permet également de moduler la fonction immunitaire en favorisant l'action des cytokines. Lorsque du colostrum est présent dans la composition, la lactoferrine augmente son action.

L'hydrolysat de la caséine est un hydrolysat de la caséine αₛ₁, à savoir l'α-casozépine. L'α-casozépine est le décapeptide de SEQ ID NO. 1. Des exemples de fragments de l'α-casozépine sont le nonapeptide de SEQ ID NO. 2, l'octapeptide de SEQ ID NO. 3, l'heptapeptide de SEQ ID NO. 4, l'hexapeptide de SEQ ID NO.5 et le pentapeptide de SEQ ID NO. 6. L'α-casozépine et les fragments mentionnés ci-dessus possèdent des propriétés tranquillisantes. Ils accroissent l'activité de l'acide gamma-aminobutyrique (GABA), un neurotransmetteur ayant une action inhibitrice de l'anxiété et des propriétés relaxantes et favorisent la diminution de la pression sanguine.

La composition comprend avantageusement de 5 à 50 % en poids, de préférence de 10 à 30 % en poids, plus préférentiellement de 15 à 20 % en poids, de glycoprotéine liant le fer, et de préférence de lactoferrine, par rapport au poids total de la composition.

La composition peut comprendre de 2 à 30 % en poids, de préférence de 3 à 20 % en poids, plus préférentiellement de 5 à 10 % en poids, d'hydrolysat de la caséine, par rapport au poids total de la composition.

Le ratio massique de glycoprotéine liant le fer par rapport à l'hydrolysat de la caséine est de 0,5 à 3, plus préférentiellement 2. Dans des modes de réalisation, le ratio massique de glycoprotéine liant le fer par rapport à l'hydrolysat de la caséine est de 0,5 à 0,6, ou de 0,6 à 0,7, ou de 0,7 à 0,8, ou de 0,8 à 0,9, ou de 0,9 à 1, ou de 1 à 2, ou de 2 à 3.

La composition peut en outre comprendre du colostrum (composé C), par exemple du colostrum bovin, ovin, équin ou porcin. De préférence, le colostrum est du colostrum bovin. Le colostrum est le lait sécrété par les mammifères femelles juste après l'accouchement. Il comprend de nombreux composants, dont des immunoglobulines IgG1, IGg2, IgM, IgA, de la lactoferrine, de l'albumine sérique, de la transferrine et de l'α-glycoprotéine acide. Le colostrum possède des propriétés énergisantes. Il permet aux cellules de combattre les agressions intrinsèques et extrinsèques et de conserver leur vitalité plus longtemps. Il renforce les membranes cellulaires.

La composition peut par exemple comprendre de 15 à 70 % en poids, de préférence de 20 à 50 % en poids, plus préférentiellement de 25 à 40 % en poids, de matière sèche de colostrum, par rapport au poids total de la composition.

Le ratio massique de matière sèche de colostrum par rapport à l'hydrolysat de la caséine est avantageusement de 0,5 à 10, de préférence de 1 à 6, plus préférentiellement 4. Dans des modes de réalisation, le ratio massique de colostrum par rapport à l'hydrolysat de la caséine est de 0,5 à 0,6, ou de 0,6 à 0,7, ou de 0,7 à 0,8, ou de 0,8 à 0,9, ou de 0,9 à 1, ou de 1 à 2, ou de 2 à 3, ou de 3 à 4, ou de 4 à 5, ou de 5 à 6, ou de 6 à 7, ou de 7 à 8, ou de 8 à 9, ou de 9 à 10.

Il est entendu ici que les quantités et ratios massiques de matière sèche de colostrum ci-dessus se rapportent au poids total des composés de la matière sèche du colostrum à l'exclusion des composés qui sont également apportés dans la composition par un apport spécifique. Ainsi, le poids de matière sèche de colostrum ne prend pas en compte le poids de la lactoferrine apporté par le colostrum (qui est comptabilisé séparément). Toutefois, dans la composition selon l'invention, la quantité de lactoferrine apportée par le colostrum est généralement négligeable par rapport à la quantité totale de matière sèche de colostrum.

Dans des modes de réalisation, la composition comprend en outre un précurseur de l'oxyde nitrique (composé D), de préférence de la L-arginine. L'oxyde nitrique est une molécule signal ayant pour fonction la dilatation des vaisseaux sanguins. Le précurseur de l'oxyde nitrique facilite la migration des autres composés de la composition dans la peau. Notamment, lorsque du colostrum est présent dans la composition, le précurseur de l'oxyde nitrique permet de faciliter la pénétration des composés du colostrum dans la peau. L'arginine est de plus capable de stimuler les cellules pour qu'elles produisent plus de protéines telles que le collagène et l'élastine et qu'elles puissent réparer le patrimoine héréditaire endommagé.

La composition peut comprendre de 10 à 50 % en poids, de préférence de 15 à 40 % en poids, plus préférentiellement de 20 à 30 % en poids, du précurseur de l'oxyde nitrique, par rapport au poids total de la composition.

Le ratio massique du précurseur de l'oxyde nitrique par rapport à l'hydrolysat de la caséine est avantageusement de 0,1 à 10, de préférence de 1 à 5, plus préférentiellement 3. Dans des modes de réalisation, le ratio massique du précurseur de l'oxyde nitrique par rapport à l'hydrolysat de la caséine est de 0,1 à 0,2, ou de 0,2 à 0,3, ou de 0,3 à 0,4, ou de 0,4 à 0,5, ou de 0,5 à 0,6, ou de 0,6 à 0,7, ou de 0,7 à 0,8, ou de 0,8 à 0,9, ou de 0,9 à 1, ou de 1 à 2, ou de 2 à 3, ou de 3 à 4, ou de 4 à 5, ou de 5 à 6, ou de 6 à 7, ou de 7 à 8, ou de 8 à 9, ou de 9 à 10.

Un antioxydant peut également être présent dans la composition (composé E), de préférence un caroténoïde. De manière particulièrement préférée, l'antioxydant est l'astaxanthine. L'astaxanthine possède une action anti-inflammatoire et antioxydante et protège la double membrane mitochondriale en améliorant ses fonctions et en augmentant par conséquent la capacité de la mitochondrie à produire de l'énergie. Elle peut voyager à travers tout l'organisme, apportant une action antioxydante et anti-inflammatoire à tous les organes et tissus. Elle permet également le renforcement de la membrane cellulaire.

Alternativement, ou en combinaison avec un caroténoïde, par exemple l'astaxanthine, l'antioxydant peut être un rétinoïde tel que le rétinol, la vitamine C (l'acide ascorbique et ses sels) et/ou la vitamine E.

De préférence, l'antioxydant est présent, par rapport au poids total de la composition, en une quantité de 2 à 30 % en poids, de préférence de 3 à 20 % en poids, plus préférentiellement de 5 à 10 % en poids.

Le ratio massique de l'antioxydant par rapport à l'hydrolysat de la caséine est avantageusement de 0,1 à 5, de préférence de 0,5 à 3, plus préférentiellement 1. Dans des modes de réalisation, le ratio massique de l'antioxydant par rapport à l'hydrolysat de la caséine est de 0,1 à 0,2, ou de 0,2 à 0,3, ou de 0,3 à 0,4, ou de 0,4 à 0,5, ou de 0,5 à 0,6, ou de 0,6 à 0,7, ou de 0,7 à 0,8, ou de 0,8 à 0,9, ou de 0,9 à 1, ou de 1 à 2, ou de 2 à 3, ou de 3 à 4, ou de 4 à 5.

La composition peut en outre comprendre de la sérotonine ou un de ses précurseurs (composé F). De préférence, la composition comprend un précurseur de la sérotonine, et plus préférentiellement du tryptophane, encore plus préférentiellement du L-tryptophane. La sérotonine et ses précurseurs, dont le tryptophane, ont des propriétés tranquillisantes et jouent un rôle la dépression, l'anxiété, l'humeur et le contrôle de l'appétit. L'utilisation d'un précurseur de la sérotonine tel que le tryptophane est particulièrement avantageuse car la sérotonine est incapable de traverser la barrière hémato-encéphalique alors qu'un précurseur tel que le tryptophane en est capable. De plus, le tryptophane permet de potentialiser l'action de l'hydrolysat de caséine. Il participe à la formation des lymphocytes actifs du système de défense de l'organisme et agit au niveau du stress cellulaire.

La composition peut comprendre de 2 à 30 % en poids de préférence de 3 à 20 % en poids, plus préférentiellement de 5 à 10 % en poids, de sérotonine ou d'un de ses précurseurs, par rapport au poids total de la composition.

Le ratio massique de sérotonine ou d'un de ses précurseurs par rapport à l'hydrolysat de la caséine est avantageusement de 0,1 à 5, de préférence de 0,5 à 3, plus préférentiellement 1. Dans des modes de réalisation, le ratio massique de sérotonine ou d'un de ses précurseurs par rapport à l'hydrolysat de la caséine est de 0,1 à 0,2, ou de 0,2 à 0,3, ou de 0,3 à 0,4, ou de 0,4 à 0,5, ou de 0,5 à 0,6, ou de 0,6 à 0,7, ou de 0,7 à 0,8, ou de 0,8 à 0,9, ou de 0,9 à 1, ou de 1 à 2, ou de 2 à 3, ou de 3 à 4, ou de 4 à 5.

Dans des modes de réalisation, la composition comprend en outre au moins une charge minérale, de préférence une argile, plus préférentiellement choisie parmi l'illite, la montmorillonite, la kaolinite, ou un mélange de celles-ci. De manière particulièrement préférée, la composition comprend un mélange d'illite, de montmorillonite et de kaolinite.

Lorsque la composition comprend une charge minérale, cette dernière est avantageusement présente, par rapport au poids total de la composition, en une quantité de 5 à 80 % en poids, de préférence de 10 à 50% en poids.

Le ratio massique de charge minérale par rapport à l'hydrolysat de la caséine est avantageusement de 0,1 à 10, de préférence de 0,5 à 5. Dans des modes de réalisation, le ratio massique d'argile par rapport à l'hydrolysat de la caséine est de 0,1 à 0,2, ou de 0,2 à 0,3, ou de 0,3 à 0,4, ou de 0,4 à 0,5, ou de 0,5 à 0,6, ou de 0,6 à 0,7, ou de 0,7 à 0,8, ou de 0,8 à 0,9, ou de 0,9 à 1, ou de 1 à 2, ou de 2 à 3, ou de 3 à 4, ou de 4 à 5, ou de 5 à 6, ou de 6 à 7, ou de 7 à 8, ou de 8 à 9, ou de 9 à 10.

Dans des modes de réalisation, la composition comprend les composés A, B et C décrits ci-dessus.

Dans des modes de réalisation, la composition comprend les composés A, B et D décrits ci-dessus.

Dans des modes de réalisation, la composition comprend les composés A, B et E décrits ci-dessus.

Dans des modes de réalisation, la composition comprend les composés A, B et F décrits ci-dessus.

Dans des modes de réalisation, la composition comprend les composés A, B, C et D décrits ci-dessus.

Dans des modes de réalisation, la composition comprend les composés A, B, C et E décrits ci-dessus.

Dans des modes de réalisation, la composition comprend les composés A, B, C et F décrits ci-dessus.

Dans des modes de réalisation, la composition comprend les composés A, B, D et E décrits ci-dessus.

Dans des modes de réalisation, la composition comprend les composés A, B, D et F décrits ci-dessus.

Dans des modes de réalisation, la composition comprend les composés A, B, E et F décrits ci-dessus.

Dans des modes de réalisation, la composition comprend les composés A, B, C, D et E décrits ci-dessus.

Dans des modes de réalisation, la composition comprend les composés A, B, C, D et F décrits ci-dessus.

Dans des modes de réalisation, la composition comprend les composés A, B, C, E et F décrits ci-dessus.

Dans des modes de réalisation, la composition comprend les composés A, B, D, E et F décrits ci-dessus.

Dans des modes de réalisation, la composition comprend les composés A, B, C, D, E et F décrits ci-dessus.

Dans des modes de réalisation, la composition consiste essentiellement, voire consiste, en les composés A, B, et le cas échéant C et/ou D et/ou E et/ou F, et optionnellement une charge minérale et/ou un véhicule et/ou un ou des additifs de formulation (épaississant, pigment ou colorant, etc.).

La composition est de préférence formulée sous forme de poudre. Alternativement, elle peut être formulée sous une forme liquide, sous la forme d'un gel, par exemple d'un gel huileux, d'une crème, de revêtement sur un patch, ou de granulés, ces formes étant de préférence fabriquées à partir d'une poudre de composition qui est ensuite formulée avec un véhicule et des additifs de formulation.

La composition est de préférence une composition cosmétologique ou une composition dermatologique.

### Procédé de préparation

L'invention concerne également un procédé de préparation de la composition décrite ci-dessus, comprenant le mélange, en une ou plusieurs étapes, de la glycoprotéine liant le fer et de l'hydrolysat de la caséine, et optionnellement du colostrum et/ou du précurseur de l'oxyde nitrique et/ou de l'antioxydant et/ou de la sérotonine ou précurseur de celle-ci et/ou de l'argile et/ou de l'excipient.

De préférence, la glycoprotéine liant le fer et/ou l'hydrolysat de la caséine et/ou le colostrum et/ou le précurseur de l'oxyde nitrique, et/ou l'antioxydant et/ou la sérotonine ou un de ses précurseurs et/ou l'argile sont intégrées sous forme de poudre.

De manière avantageuse, le mélange est effectué à une température allant de 12°C à 25°C, plus préférentiellement à une à une température allant de 15°C à 20°C, encore plus préférentiellement à une à une température allant de 17°C à 19°C. De telles températures permettent de préserver les qualités des composés et d'éviter leur dégradation.

Le mélange est de préférence effectué sous agitation lente.

### Utilisations

L'invention concerne également un procédé de traitement cosmétique de la peau. Ce procédé comprend l'application topique d'une composition telle que définie ci-dessus.

Dans certains modes de réalisation, le traitement cosmétique comprend ou consiste en le comblement des rides, la diminution des poches sous les yeux, la réduction de la couleur des cernes, la réduction des tâches de vieillissement, l'augmentation de l'élasticité de la peau et/ou l'atténuation des vergetures.

De préférence, la composition est appliquée pour le traitement cosmétique en une quantité allant de 0,1 mg/cm² de peau à 50 mg/cm² de peau, de préférence de 0,5 mg/cm² de peau à 20 mg/cm² de peau, encore plus préférentiellement de 0,8 mg/cm² de peau à 10 mg/cm² de peau. Dans des modes de réalisation, la composition est appliquée en une quantité de 0,1 à 0,3 mg/cm² de peau, ou de 0,3 à 0,5 mg/cm² de peau, ou de 0,5 à 0,8 mg/cm² de peau, ou 0,8 à 1 mg/cm² de peau, ou de 1 à 1,5 mg/cm² de peau, ou de 1,5 à 2 mg/cm² de peau, ou de 2 à 2,5 mg/cm² de peau, ou de 2,5 à 3 mg/cm² de peau, ou de 3 à 3,5 mg/cm² de peau, ou de 3,5 à 4 mg/cm² de peau, ou de 4 à 4,5 mg/cm² de peau, ou de 4,5 à 5 mg/cm² de peau, ou de 5 à 6 mg/cm² de peau, ou de 6 à 7 mg/cm² de peau, ou de 7 à 8 mg/cm² de peau, ou de 8 à 9 mg/cm² de peau, ou de 9 à 10 mg/cm² de peau, ou de 10 à 15 mg/cm² de peau, ou de 15 à 20 mg/cm² de peau, ou de 20 à 25 mg/cm² de peau, ou de 25 à 30 mg/cm² de peau, ou de 30 à 35 mg/cm² de peau, ou de 35 à 40 mg/cm² de peau, ou de 40 à 45 mg/cm² de peau, ou de 45 à 50 mg/cm² de peau.

La composition peut être notamment appliquée une fois tous les deux jours, ou une fois par jour, ou deux fois par jour, ou trois fois par jour.

Un autre aspect de l'invention est une composition telle que décrite ci-dessus pour son utilisation dans le traitement des maladies dermatologiques.

Des exemples de maladies dermatologiques sont l'atrophie cutanée, l'eczéma et/ou les chéloïdes. Le traitement de l'atrophie cutanée est préféré.

De préférence, la composition est administrée par voie topique. Dans ce cas, la composition est avantageusement appliquée pour le traitement dermatologique en une quantité allant de 0,1 mg/cm² de peau à 50 mg/cm² de peau, de préférence de 0,5 mg/cm² de peau à 20 mg/cm² de peau, encore plus préférentiellement de 0,8 mg/cm² de peau à 10 mg/cm² de peau. Dans des modes de réalisation, la composition est appliquée en une quantité de 0,1 à 0,3 mg/cm² de peau, ou de 0,3 à 0,5 mg/cm² de peau, ou de 0,5 à 0,8 mg/cm² de peau, ou 0,8 à 1 mg/cm² de peau, ou de 1 à 1,5 mg/cm² de peau, ou de 1,5 à 2 mg/cm² de peau, ou de 2 à 2,5 mg/cm² de peau, ou de 2,5 à 3 mg/cm² de peau, ou de 3 à 3,5 mg/cm² de peau, ou de 3,5 à 4 mg/cm² de peau, ou de 4 à 4,5 mg/cm² de peau, ou de 4,5 à 5 mg/cm² de peau, ou de 5 à 6 mg/cm² de peau, ou de 6 à 7 mg/cm² de peau, ou de 7 à 8 mg/cm² de peau, ou de 8 à 9 mg/cm² de peau, ou de 9 à 10 mg/cm² de peau, ou de 10 à 15 mg/cm² de peau, ou de 15 à 20 mg/cm² de peau, ou de 20 à 25 mg/cm² de peau, ou de 25 à 30 mg/cm² de peau, ou de 30 à 35 mg/cm² de peau, ou de 35 à 40 mg/cm² de peau, ou de 40 à 45 mg/cm² de peau, ou de 45 à 50 mg/cm² de peau.

La composition peut être appliquée une fois tous les deux jours, ou une fois par jour, ou deux fois par jour, ou trois fois, quatre, ou cinq fois par jour.

### EXEMPLES

Les exemples suivants illustrent l'invention sans la limiter.

### Exemple 1

### Préparation des compositions

Une composition (Produit A) a été préparée par mélange des composants suivants sous forme de poudre :

| **Composants** | **Masse (g) pour 12 g de composition** | **Fournisseur du composant** |
|---|---|---|
| Colostrum bovin contenant 30% d'immunoglobulines actives G | 4 | INGREDIA France |
| L-arginine | 3 | UNIPEX/ Ajinomoto |
| Lactoferrine | 2 | INGREDIA France |
| Alpha-casozépine | 1 | INGREDIA France |
| Astaxanthine | 1 | UNIPEX/ Ajinomoto |
| Tryptophane | 1 | UNIPEX/ Ajinomoto |

Une composition (produit P2) a ensuite été préparée en diluant 15% du produit A dans un gel de carboxyméthylcellulose (gel CMC).

### Préparation et traitement des explants

Neuf explants d'un diamètre de 12 mm (±1mm) ont été préparés à partir d'une plastie abdominale d'une femme caucasienne de 50 ans. Les explants ont été mis en survie en milieu BEM (BIO-EC's Explants Medium) à 37°C en atmosphère humide enrichie de 5 % de CO₂.

Les explants ont été répartis en 3 lots comme suit :

| **Lot** | **Traitement** | **Nombre d'explants** | **Jour de l'arrêt de l'expérimentation** |
|---|---|---|---|
| T0 (témoin plastie) | Aucun | 3 | J0 |
| T (témoin non traité) | Aucun | 3 | J12 |
| LP2 | Produit P2 | 3 | J12 |

J0 désigne le jour du début de l'expérimentation et Jn désigne le énième jour suivant le début de l'expérimentation.

A J0, J2, J3, J6, J8 et J10, le produit P2 a été appliqué en topique à l'aide d'une spatule en une quantité de 2 mg/cm² sur les explants du lot correspondant LP2. Les explants des lots témoins (T0 et T) n'ont reçu aucun traitement à l'exception du renouvellement du milieu de culture. A J2, J3, J6, J8 et J10, le milieu de culture a été renouvelé à moitié (1 mL de milieu BEM par puits).

A J0, les trois explants du lot T0 ont été prélevés et coupés en deux. Une des deux parties a été fixée dans une solution de formol tamponnée, et l'autre a été congelée à -80°C. A J12, les explants de chaque lot T et LP2 ont été prélevés et traités de la même façon.

### Méthodes de caractérisation des explants

### - Traitements histologiques et contrôle de la viabilité cellulaire

Après 24h dans le formol tamponné, les prélèvements ont été déshydratés et imprégnés en paraffine à l'aide d'un automate de déshydratation Leica TP 1020. Ils ont été mis en bloc à l'aide d'une station d'enrobage Leica EG 1160. Des coupes de 5 µm ont été réalisées à l'aide d'un microtome type Minot, Leica RM 2125 et montées sur des lames de verre histologiques Superfrost^{®}. Les observations microscopiques ont été réalisées en microscopie optique, à l'aide d'un microscope Leica type DMLB ou Olympus BX43. Les prises de vue ont été réalisées avec une caméra Olympus DP72 et le logiciel Cell^D.

La viabilité cellulaire a été observée sur coupes en paraffine après coloration au trichrome de Masson, variante de Goldner. Elle a été évaluée par un examen microscopique

### - Mesure de l'épaisseur de l'épiderme par analyse d'images

Une analyse des images obtenues après coloration au trichrome de Masson a été effectuée dans le but de mesurer l'épaisseur de l'épiderme. Pour cela, les mesures ont été réalisées sur trois régions d'intérêt par image, soit 27 mesures par lot.

### Résultats

A J12, la viabilité cellulaire du lot témoin T est assez bonne dans l'épiderme et bonne dans le derme.

Sur les explants traités avec le produit P2, on observe une augmentation de l'épaisseur de l'épiderme causée par une acanthose hypertrophique (augmentation de l'épaisseur de l'épiderme due à une augmentation de la taille des kératinocytes). Les cellules sont plus grosses, ce qui traduit un apport nutritionnel aux cellules accru.

Les mesures de l'épaisseur de l'épiderme sont résumées dans le tableau ci-dessous :

| Lot | T0 | T | LP2 |
|---|---|---|---|
| Jour des mesures | J0 | J12 | J12 |
| Epaisseur moyenne de l'épiderme (µm) | 28,5 | 50,3 | 66,1 |
| Ecart-type | 4,6 | 7,3 | 6,9 |

L'application du produit P2 a induit une augmentation significative de l'épaisseur de l'épiderme de 31% par rapport à l'épiderme témoin non traité (*p*<0,01).

Les cellules des explants traités avec le produit P2 sont plus grosses que les cellules des explants témoins non traités. Elles sont en bonne condition pour résister aux divers stress biologiques et environnementaux. De plus, la longévité cellulaire est également améliorée.

### Exemple 2

### Préparation des produits

Les compositions suivantes ont été préparées :
- Produit P1' : lactoferrine (poudre) diluée à une concentration finale de 15 % en poids dans un gel de carboxyméthylcellulose (gel CMC) ;
- Produit P2' : alpha-casozépine (poudre) diluée à une concentration finale de 15 % en poids dans un gel CMC ;
- Produit P3' : mélange de lactoferrine et d'alpha-casozépine (poudre) dilué à une concentration finale de 15 % en poids dans un gel CMC ;
- Produit P4' : produit A tel que décrit ci-dessus dans l'exemple 1 (poudre), dilué à une concentration finale de 15 % en poids dans un gel CMC.

Les produits P3' et P4' sont des compositions selon l'invention, les produits P1' et P2' sont des exemples comparatifs.

### Préparation et traitement des explants

18 explants d'un diamètre de 12 mm (±1mm) ont été préparés à partir d'une plastie abdominale d'une femme caucasienne de 45 ans. Les explants ont été mis en survie en milieu BEM (BIO-EC's Explants Medium) à 37°C en atmosphère humide enrichie de 5 % de CO₂.

Les explants ont été répartis en 6 lots comme suit :

| **Lot** | **Traitement** | **Nombre d'explants** | **Jour de l'arrêt de l'expérimentation** |
|---|---|---|---|
| T0' (témoin plastie) | Aucun | 3 | J0 |
| T' (témoin non traité) | Aucun | 3 | J18 |
| LP1' | Produit P1' | 3 | J18 |
| LP2' | Produit P2' | 3 | J18 |
| LP3' | Produit P3' | 3 | J18 |
| LP4' | Produit P4' | 3 | J18 |

J0 désigne le jour du début de l'expérimentation et Jn désigne le énième jour suivant le début de l'expérimentation.

A J0, J2, J3, J6, J8, J10, J13, J15 et J17, les produits P1', P2', P3' et P4' ont été appliqués en topique en une quantité de 2 µL par explant et étalés à l'aide d'une spatule, sur les explants des lots LP1', LP2', LP3' et LP4' respectivement. Les explants des lots témoins (T0' et T') n'ont reçu aucun traitement à l'exception du renouvellement du milieu de culture. A J2, J3, J6, J8, J10, J13, J15 et J17, le milieu de culture a été renouvelé à moitié (1 mL de milieu BEM par puits).

A J0, les trois explants du lot T0' ont été prélevés et coupés en deux. Une des deux parties a été fixée dans une solution de formol tamponnée, et l'autre a été congelée et conservée à -80°C. A J18, les explants de chaque lot T', LP1', LP2', LP3' et LP4' ont été prélevés et traités de la même façon.

### Méthodes de caractérisation des explants

Le contrôle de la viabilité cellulaire et la mesure de l'épaisseur de l'épiderme ont été réalisés tel que cela est décrit dans l'exemple 1.

L'examen de la morphologie générale a été réalisé de la même manière que l'examen de la viabilité cellulaire, c'est-à-dire que la morphologie générale a été évaluée sur coupes en paraffine après coloration au trichrome de Masson variante de Goldner, par un examen microscopique.

### Résultats

A J0, sur le lot témoin plastie T0', la viabilité cellulaire est bonne dans l'épiderme et bonne dans le derme. Le relief de la jonction dermo-épidermique (JDE) est assez net et la densité du derme est nette.

A J18, sur le lot témoin non traité T', la viabilité cellulaire est nettement altérée dans l'épiderme et très légèrement altérée dans le derme. Le relief de la jonction dermo-épidermique (JDE) est modéré et la densité du derme est faible.

Les effets suivants sur la morphologie générale des explants ont été observés à J18 suite à l'application des produits P1', P2', P3' et P4', par rapport au lot témoin T' à J18.

L'application du produit P1' induit une légère amélioration de la viabilité cellulaire dans l'épiderme et une très légère amélioration dans le derme. De plus, il induit une légère augmentation de l'épaisseur de l'épiderme causée par une acanthose hypertrophique. Enfin, il induit une légère augmentation de la densité du derme.

L'application du produit P2' induit une très légère amélioration de la viabilité cellulaire dans le derme, mais aucune modification dans l'épiderme. De plus, il induit une légère augmentation de l'épaisseur de l'épiderme causée par une acanthose hypertrophique. Enfin, il induit une légère augmentation de la densité du derme.

L'application du produit P3' induit une légère amélioration de la viabilité cellulaire dans l'épiderme et une très légère amélioration dans le derme. De plus, il induit une augmentation modérée de l'épaisseur de l'épiderme causée par une acanthose hypertrophique. Enfin, il induit une légère augmentation de la densité du derme.

L'application du produit P4' induit une amélioration modérée de la viabilité cellulaire dans l'épiderme et une très légère amélioration dans le derme. De plus, il induit une augmentation modérée de l'épaisseur de l'épiderme causée par une acanthose hypertrophique. Enfin, il induit une augmentation modérée de la densité du derme.

Les mesures de l'épaisseur de l'épiderme sont résumées dans le tableau ci-dessous :

| Lot | T0' | T' | LP1' | LP2' | LP3' | LP4' |
|---|---|---|---|---|---|---|
| Jour des mesures | J0 | J18 | J18 | J18 | J18 | J18 |
| Epaisseur moyenne de l'épiderme (µm) | 33,6 | 32,5 | 37,5 | 38,6 | 41,1 | 41,5 |
| Ecart-type | 4,8 | 6,2 | 4,9 | 5,0 | 6,7 | 7,7 |
| Augmentation de l'épaisseur de l'épiderme par rapport au lot T' à J18 (%) | - | - | 15* | 19* | 26* | 28* |

| | | | | | | |
|---|---|---|---|---|---|---|
| * = valeur significative, avec *p*<0.01 | | | | | | |

L'application du produit P3' induit une augmentation de l'épaisseur de l'épiderme nettement supérieure à celle obtenue avec les produits P1' et P2'. Le produit P3' possède donc une meilleure activité anti-âge que les produits P1' et P2'. De plus, par rapport au produit P2', le produit P3' améliore la viabilité cellulaire.

L'application du produit P4' induit également une augmentation de l'épaisseur de l'épiderme nettement supérieure à celle obtenue avec les produits P1' et P2'. Le produit P4' possède donc une meilleure activité anti-âge que les produits P1' et P2'. En outre, par rapport aux produits P1', P2' et P3', l'application du produit P4' améliore la viabilité cellulaire ainsi que la densité du derme.

## Revendications

1. Composition comprenant une glycoprotéine liant le fer et un hydrolysat de la caséine, l'hydrolysat de la caséine étant l'α-casozépine ;
dans laquelle le ratio massique de glycoprotéine liant le fer par rapport à l'α-casozépine est de 0,5 à 3.

2. Composition selon la revendication 1, dans laquelle la glycoprotéine liant le fer est la lactoferrine.

3. Composition selon l'une des revendications 1 à 2, comprenant en outre :
- du colostrum, de préférence du colostrum bovin, du colostrum ovin, du colostrum équin ou du colostrum porcin, encore plus préférentiellement du colostrum bovin ; et / ou
- un précurseur de l'oxyde nitrique, de préférence de la L-arginine ;
- un antioxydant, de préférence un caroténoïde, plus préférentiellement de l'astaxanthine ; et/ou
- de la sérotonine ou un de ses précurseurs, qui de préférence est le tryptophane.

4. Composition selon l'une des revendications 1 à 3, comprenant, en poids de matière sèche :
- de 2 à 30 %, de préférence de 10 à 30 % de glycoprotéine liant le fer ; et/ou
- de 2 à 30 %, de préférence de 3 à 20 % d'hydrolysat de caséine ; et/ou
- de 15 à 70 %, de préférence de 20 à 50 % de colostrum ; et/ou
- de 10 à 50 %, de préférence de 15 à 40 % de précurseur d'oxyde nitrique ; et/ou
- de 2 à 30 %, de préférence de 3 à 20 % d'antioxydant ; et/ou
- de 2 à 30 %, de préférence de 3 à 20 % de sérotonine ou précurseur de celle-ci.

5. Composition selon l'une des revendications 1 à 4, comprenant, en poids de matière sèche :
- de 2 à 30 %, de préférence de 10 à 30 % de lactoferrine ; et/ou
- de 2 à 30 %, de préférence de 3 à 20 % d'α-casozépine ; et/ou
- de 15 à 70 %, de préférence de 20 à 50 % de colostrum ; et/ou
- de 10 à 50 %, de préférence de 15 à 40 % de L-arginine ; et/ou
- de 2 à 30 %, de préférence de 3 à 20 % d'astaxanthine ; et/ou
- de 2 à 30 %, de préférence de 3 à 20 % de tryptophane.

6. Composition selon l'une des revendications 1 à 5, comprenant, en poids de matière sèche :
- de 15 à 20 % de lactoferrine ;
- de 5 à 10 % d'α-casozépine ;
- de 25 à 45 % de colostrum ;
- de 20 à 30 % de L-arginine ;
- de 5 à 10 % d'astaxanthine ; et
- de 5 à 10 % de tryptophane.

7. Procédé de traitement cosmétique de la peau, comprenant l'application topique d'une composition selon l'une des revendications 1 à 6.

8. Procédé selon la revendication 7, dans lequel le traitement cosmétique de la peau est le comblement des rides, la diminution des poches sous les yeux, la réduction de la couleur des cernes, la réduction des tâches de vieillissement, l'augmentation de l'élasticité de la peau et/ou l'atténuation des vergetures.

9. Composition selon l'une des revendications 1 à 6, pour son utilisation dans le traitement des maladies dermatologiques.

10. Composition selon la revendication 9, pour son utilisation dans le traitement de l'atrophie cutanée, l'eczéma et/ou les chéloïdes.

## Patentansprüche

1. Zusammensetzung, umfassend ein eisenbindendes Glycoprotein und ein Caseinhydrolysat, wobei das Caseinhydrolysat α-Casozepin ist;
wobei das Massenverhältnis von eisenbindendem Glycoprotein mit Bezug auf α-Casozepin von 0,5 bis 3 ist.

2. Zusammensetzung nach Anspruch 1, wobei das eisenbindende Glycoprotein Lactoferin ist.

3. Verfahren nach einem der Ansprüche 1 bis 2, umfassend außerdem:
- Kolostrum, vorzugsweise Rinderkolostrum, Schafskolostrum, Pferdekolostrum oder Schweinekolostrum, noch bevorzugter Rinderkolostrum; und/oder
- einen Vorläufer von Stickoxid, vorzugsweise L-arginin;
- ein Antioxidationsmittel, vorzugsweise ein Carotenoid, noch bevorzugter Astaxanthin; und/oder
- Serotonin oder einen seiner Vorläufer, wobei es sich vorzugsweise um Tryptohpan handelt.

4. Zusammensetzung nach einem der Ansprüche 1 bis 3, umfassend in Trockenmassegewicht:
- von 2 bis 30 %, vorzugsweise von 10 bis 30 % eisenbindendes Glycoprotein;
und/oder
- von 2 bis 30 %, vorzugsweise von 3 bis 20 % Caseinhydrolysat; und/oder
- von 15 bis 70 %, vorzugsweise von 20 bis 50 % Kolostrum; und/oder
- von 10 bis 50 %, vorzugsweise von 15 bis 40 % Stickstoffvorläufer; und/oder
- von 2 bis 30 %, vorzugsweise von 3 bis 20 % Antioxidationsmittel; und/oder
- von 2 bis 30 %, vorzugsweise von 3 bis 20 % Serotonin oder Vorläufer desselben.

5. Zusammensetzung nach einem der Ansprüche 1 bis 4, umfassend in Trockenmassegewicht:
- von 2 bis 30 %, vorzugsweise von 10 bis 30 % Lactoferin; und/oder
- von 2 bis 30 %, vorzugsweise von 3 bis 20 % α-Casozepin; und/oder
- von 15 bis 70 %, vorzugsweise von 20 bis 50 % Kolostrum; und/oder
- von 10 bis 50 %, vorzugsweise von 15 bis 40 % L-arginin; und/oder
- von 2 bis 30 %, vorzugsweise von 3 bis 20 % Astaxanthin; und/oder
- von 2 bis 30 %, vorzugsweise von 3 bis 20 % Tryptophan.

6. Zusammensetzung nach einem der Ansprüche 1 bis 5, umfassend in Trockenmassegewicht:
- von 15 bis 20 % Lactoferin;
- von 5 bis 10 % α-Casozepin;
- von 25 bis 45 % Kolostrum;
- von 20 bis 30 % L-arginin;
- von 5 bis 10 % Astaxanthin; und
- von 5 bis 10 % Tryptophan.

7. Verfahren zur kosmetischen Behandlung der Haut, umfassend die topische Anwendung einer Zusammensetzung nach einem der Ansprüche 1 bis 6.

8. Verfahren nach Anspruch 7, wobei die kosmetische Behandlung der Haut das Ausfüllen von Falten, die Verkleinerung der Tränensäcke unter den Augen, die Reduzierung der Farbe der Augenringe, die Reduzierung der Altersflecken, die Erhöhung der Elastizität der Haut und/oder die Linderung der Dehnungsstreifen ist.

9. Zusammensetzung nach einem der Ansprüche 1 bis 6 für ihre Verwendung bei der Behandlung der dermatologischen Krankheiten.

10. Zusammensetzung nach Anspruch 9 für ihre Verwendung bei der Behandlung der Hautatrophie, dem Ekzem und/oder den Keloiden.

## Claims

1. A composition comprising an iron-binding glycoprotein and a casein hydrolysate, the casein hydrolysate being α-casozepine;
wherein the weight ratio of iron-binding glycoprotein to α-casozepine is from 0.5 to 3.

2. The composition according to claim 1, wherein the iron-binding glycoprotein is lactoferrin.

3. The composition according to one of claims 1 to 2, further comprising:
- colostrum, preferably bovine colostrum, ovine colostrum, equine colostrum or porcine colostrum, more preferably bovine colostrum; and/or
- a precursor of nitric oxide, preferably L-arginine;
- an antioxidant, preferably a carotenoid, more preferably astaxanthin; and/or
- serotonin or one of the precursors thereof, which is preferably tryptophan.

4. The composition according to one of claims 1 to 3, comprising, by weight of dry matter:
- from 2 to 30 %, preferably 10 to 30 % of iron-binding glycoprotein; and/or
- from 2 to 30 %, preferably 3 to 20 % of casein hydrolysate; and/or
- from 15 to 70 %, preferably 20 to 50 % of colostrum; and/or
- from 10 to 50 %, preferably 15 to 40 % of nitric oxide precursor; and/or
- from 2 to 30 %, preferably 3 to 20 % of antioxidant; and/or
- from 2 to 30 %, preferably 3 to 20 % of serotonin or precursor thereof.

5. The composition according to one of claims 1 to 4, comprising, by weight of dry matter:
- from 2 to 30 %, preferably 10 to 30 % of lactoferrin; and/or
- from 2 to 30 %, preferably 3 to 20 % of α-casozepine; and/or
- from 15 to 70 %, preferably 20 to 50 % of colostrum; and/or
- from 10 to 50 %, preferably 15 to 40 % of L-arginine; and/or
- from 2 to 30 %, preferably 3 to 20 % of astaxanthin; and/or
- from 2 to 30 %, preferably 3 to 20 % of tryptophan.

6. The composition according to one of claims 1 to 5, comprising, by weight of dry matter:
- from 15 to 20 % of lactoferrin;
- from 5 to 10 % of α-casozepine;
- from 25 to 45 % of colostrum;
- from 20 to 30 % of L-arginine;
- from 5 to 10 % of astaxanthin; and
- from 5 to 10 % of tryptophan.

7. A method for cosmetic treatment of the skin, comprising the topical application of a composition according to one of claims 1 to 6.

8. The method according to claim 7, wherein the cosmetic treatment of the skin consists in filling wrinkles, reducing under-eye bags, lightening under-eye dark circles, reducing age spots, increasing skin elasticity and/or attenuating stretch marks.

9. The composition according to one of claims 1 to 6, for its use thereof in the treatment of dermatological diseases.

10. The composition according to claim 9, for use thereof in the treatment of skin atrophy, eczema and/or cheloids.
